# EUROPÄISCHE PATENTSCHRIFT

(11) **EP 0 645 991 B1**
(45) Veröffentlichungstag und Bekanntmachung des Hinweises auf die Patenterteilung: **27.10.1999**
(21) Anmeldenummer: 93912978.9
(22) Anmeldetag: 15.06.1993
(51) Int. Cl.: A61F 2/46, A61F 2/30

(54) **MEMBRANSIEGEL FÜR DIE ABDICHTUNG VON KNOCHENÖFFNUNGEN**
MEMBRANE SEAL FOR SEALING OFF APERTURES IN BONES
SCELLEMENT MEMBRANAIRE POUR BOUCHER DES ORIFICES AU NIVEAU DES OS

(30) Priorität: 15.06.1992 DE 4219564
(43) Veröffentlichungstag der Anmeldung: 05.04.1995
(73) Patentinhaber: Draenert, Klaus, Dr.med.Dr.med.habil., D-81545 München (DE)
(72) Erfinder: Draenert, Klaus, Dr.med.Dr.med.habil., D-81545 München (DE)
(86) Internationale Anmeldenummer: EP9301511
(87) Internationale Veröffentlichungsnummer: WO9325163

(56) Entgegenhaltungen:
- EP-A- 0 073 604
- EP-A- 0 093 560
- EP-A- 0 315 283
- WO-A-90/00375
- US-A- 4 997 448

## Beschreibung

Die Erfindung betrifft eine Vorrichtung zum Abdichten eines eröffneten Röhrenknochens, einer Knochenöffnung, einer Osteotomiefläche, eines Knochenfensters oder eines Frakturspaltes. Eine solche Vorrichtung ist aus der EP-A-0 073 604 bekannt. Insbesondere betrifft die Erfindung ein Membransiegel für die Abdichtung von Knochenöffnungen.

Aus der WO-A-90/00375 ist eine Vorrichtung zum Abdichten der Markhöhle eines Knochens bei der Applikation von Knochenzement bekannt, die ein elastisches Unterteil zum formschlüssigen Kontakt mit dem Knochen um die Markhöhle herum und ein starres Oberteil zum Anpressen des elastischen Unterteils an den Knochen aufweist. Diese Vorrichtung ist insbesondere dann vorteilhaft, wenn das elastische Unterteil mittels einer Knochenzementkartusche an eine im wesentlichen ebene Fläche, wie die Resektionsebene, angepreßt werden kann.

Der Erfindung liegt die Aufgabe zugrunde, eine Vorrichtung zur Abdichtung von Knochenöffnungen bereitzustellen, die vielseitig einsetzbar ist und die insbesondere auch gegenüber unebenen Knochenflächen gut abdichtet.

Diese Aufgabe wird durch die erfindungsgemäße Vorrichtung gelöst.

Die Erfindung geht dabei von dem Grundgedanken aus, eine elastische Membran vorzusehen, die um die Knochenöffnung herum derart gegenüber dem Knochen abdichtbar angeordnet werden kann, daß im Inneren des Knochens ein Vakuum aufgebaut werden kann. Zu diesem Zweck kann die elastische Membran beispielsweise über das Knochenende gestülpt und/oder an den Knochen gepreßt werden.

Vorzugsweise weist die erfindungsgemäße Membran die Form eines Fingerlings, einer Kappe oder Tasse auf. Im ungedehnten Zustand kann die Membran auch eben ausgebildet sein und kann etwa kreisförmig oder ellipsenförmig bzw. oval sein. Der freie Rand der Membran kann verdichtet oder verdickt sein. Ein derartiger Fingerling kann einfach abdichtend über das Knochenende gestülpt bzw. auf den Knochen aufgepreßt werden. Die Membran kann vorteilhafterweise aus Silikon bestehen.

Die elastische Membran kann vorzugsweise Verdickungen aufweisen, um Konturen, Einziehungen, Gruben und/oder Öffnungen (Foramina) abzudichten.

Ferner können in der elastischen Membran Sollrißstellen, Sollperforationen und/oder Öffnungen vorgesehen sein.

Am freien Rand der elastischen Membran können Einrichtungen vorgesehen sein, mittels denen die Membran gegen den Knochen abdichtbar ist. Beispielsweise kann am freien Rand der Membran eine geführte oder fest fixierte elastische oder zugfeste Zugvorrichtung vorgesehen sein. Die Membran kann zu diesem Zweck auch Zipfel aufweisen, die um die Zirkumferenz des Knochens abdichtbar verknotbar sind.

Besonders vorteilhaft ist es, wenn am freien Rand der Membran ein schlauchförmiger Hohlraum vorgesehen ist. Dieser Hohlraum kann beispielsweise mit Gas oder einer Flüssigkeit aufgeblasen werden und bildet dann eine Art Dichtung, die um die Knochenöffnung herum abdichtbar gegen den Knochen angeordnet werden kann.

Es ist auch möglich, die erfindungsgemäße Membran, insbesondere den erfindungsgemäßen Fingerling, in einer Reihe von verschiedenen, abgestuften Größen auszubilden, um auf diese Weise ein Sortiment oder Set zur Abdichtung verschieden großer und verschieden geformter Knochenöffnungen bereitzustellen.

Die Erfindung wird nachstehend anhand der Zeichnung näher erläutert. Es zeigen die Figuren 3-5 jedoch nicht die Erfindung, da dort die elastische Membran nur einen einzigen Wulst aufweist.
- Fig. 1: die erfindungsgemäße Vorrichtung in Form eines Membransiegels zur Abdichtung eines proximalen Femurendes,
- Fig. 2: einen zur Vorbereitung der Knochenzementapplikation eröffneten Femur,
- Fig. 3 und 4: eine Vorrichtung in Form eines Fingerlings mit Zipfeln, und
- Fig. 5: eine Vorrichtung in Kombination mit einer zusätzlichen Markhöhlenabdichtvorrichtung zur Verwendung bei der Applikation von Knochenzement.

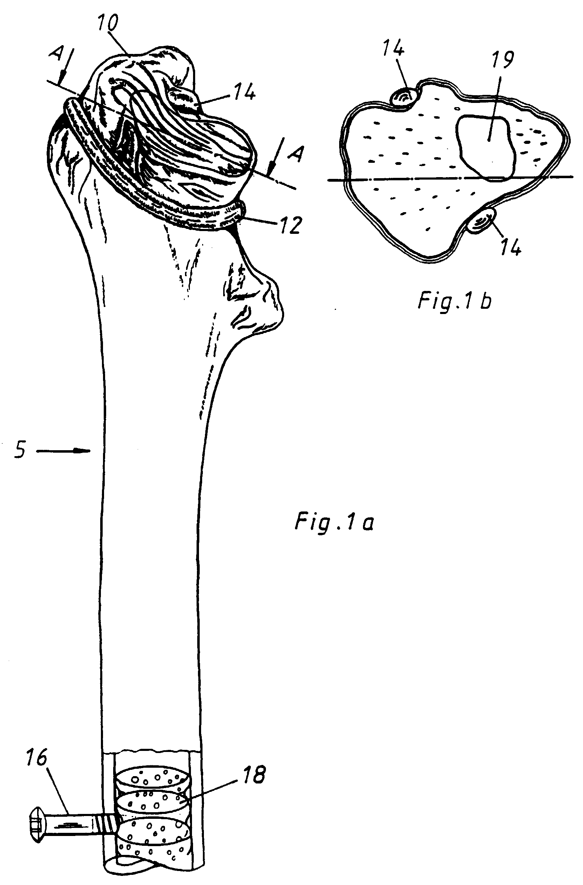

Fig. 1a zeigt das proximale Ende eines Femurs 5 mit einer erfindungsgemäßen Abdichtvorrichtung 10 in Form einer elastischen Membran. Die Abdichtvorrichtung 10 ist etwa kappenförmig über das eröffnete proximale Femurende gestülpt und weist an ihrem freien Rand einen aufblasbaren Wulst 12 auf. Der Wulst 12 bildet eine Dichtung am freien Ende der elastischen Membran der Abdichtvorrichtung 10, so daß nach dem Überstülpen der Vorrichtung 10 über das freie Knochenende im Inneren des Knochens ein zumindest partielles Vakuum aufgebaut werden kann. Ferner weist die Abdichtvorrichtung 10 etwa mittig zwei weitere Wulste 14 auf, die vorne und hinten am Rand der Membran ausgebildet sind und sich rechts bzw. links in die fossa intertrochantrica anlegen. Zum Aufbau eines Vakuums im Innern des Knochens ist eine kanülierte Saugdrainageschraube 16 vorgesehen, wie sie beispielsweise in der EP-A-305 417 beschrieben ist und die in einem Pfropfen (plug) 18 verankert ist. Fig. 1b zeigt einen Querschnitt entlang der Linie A-A von Fig. 1a, in dem die beiden wulstförmigen Verstärkungen 14 am umlaufenden Wulst 12 erkennbar sind. Mit dem Bezugszeichen 19 ist die Markhöhlenöffnung bezeichnet.

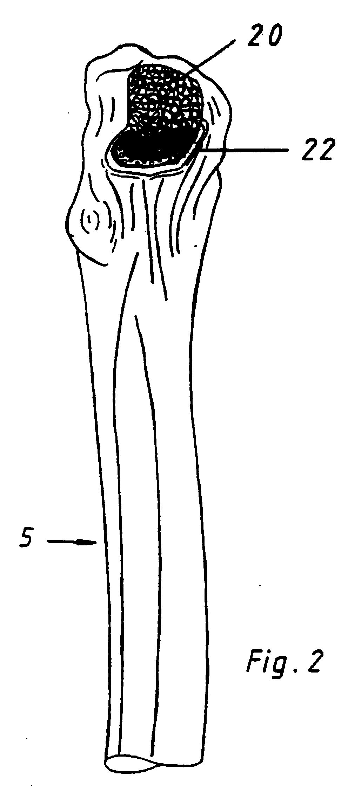

Fig. 2 zeigt eine Ansicht eines eröffneten Femurs von medial, welcher mit der erfindungsgemäßen Abdichtvorrichtung abgedichtet werden kann. Mit dem Bezugszeichen 20 ist die laterale Schnittfläche mit den kreisförmig angedeuteten Spongiosaöffnungen und mit dem Bezugszeichen 22 die Schnittfläche des Schenkelhalses bezeichnet.

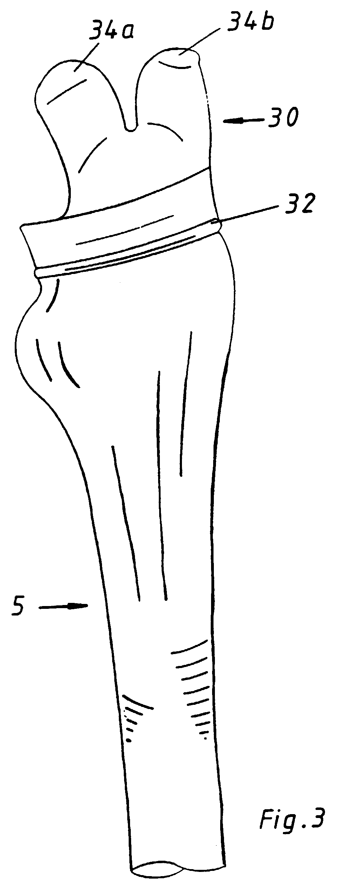

Fig. 3 zeigt den Femur um etwa 30° gegenüber Fig. 2 gedreht von ventro-medial. Auf den Femur 5 ist eine Abdichtvorrichtung 30 in Form eines Fingerlings mit einem umlaufenden Wulst 32 und zwei Zipfeln 34a und 34b dargestellt, welche über den eröffneten Femur gestülpt ist.

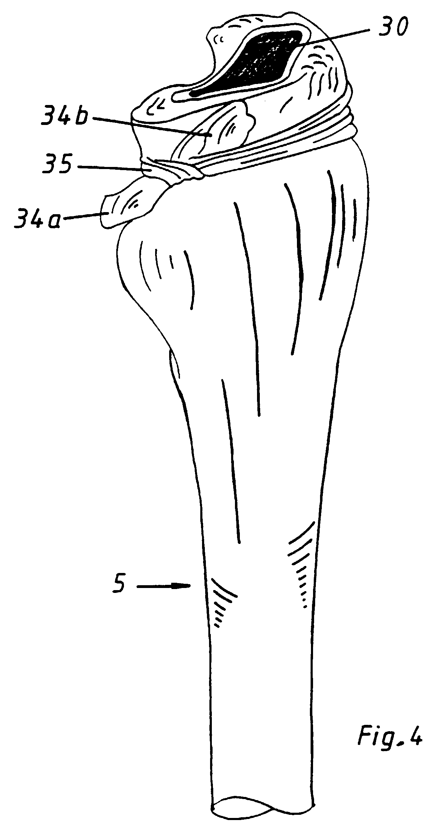

Fig. 4 zeigt den nächsten Schritt, wobei die beiden Zipfel 34a und 34b um die Zirkumferenz des Femur mittels eines medial angeordneten Knotens 35 verknotet sind und den Rand der Abdichtvorrichtung gegenüber dem Knochen abdichten. Ferner ist in Fig. 4 die Abdichtvorrichtung 30 in Form einer über die Knochenöffnung gespannten Membran ersichtlich.

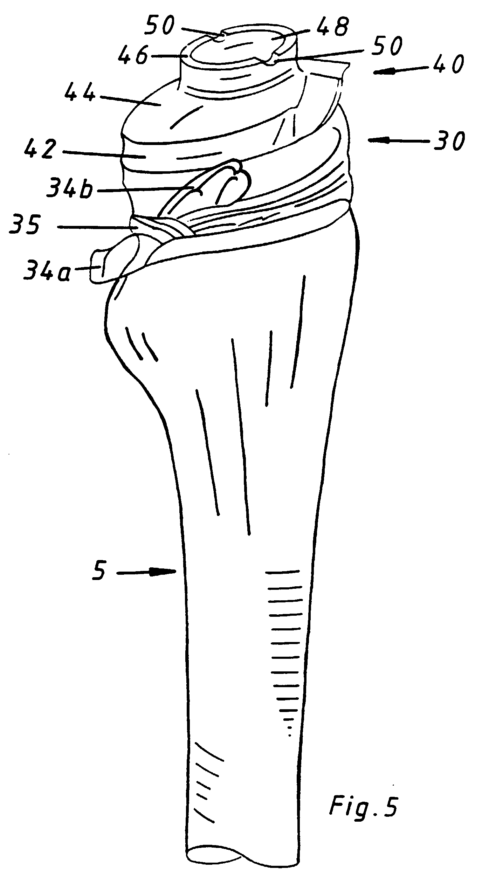

In Fig. 5 ist der nächste Schritt dargestellt, wobei auf die elastische Membran der Abdichtvorrichtung 30 eine weitere Abdichtvorrichtung 40 aufgesetzt ist, wie sie beispielsweise in der WO-A-90/00375 beschrieben ist. Die Abdichtvorrichtung 40 weist ein elastisches Unterteil 42 und ein starres Oberteil 44 mit einem üblicherweise runden oberen Rand 46 auf. Eine innere Anlagefläche 48 im Oberteil 44 der Abdichtvorrichtung 40 ist so geformt, daS sie das vordere Ende einer Knochenzementkartusche formschlüssig aufnehmen kann; sie weist die entsprechende Form wie die Spitze der (nicht dargestellten) Kartusche auf und verjüngt sich nach unten in der Regel etwa konusförmig. Die nicht rotationssymmetrisch zulaufende innere Anlagefläche 48 der Vorrichtung 40 kann eine ovale untere Austrittsöffnung aufweisen, deren Form der Austrittsöffnung der Kartusche entspricht. Am oberen Rand 46 des Oberteils 44 sind zwei Ausnehmungen 50 vorgesehen, in die Zapfen der Kartusche eingreifen können, um die Kartusche verdrehfest in der Abdichtvorrichtung 40 zu halten.

Durch die Abdichtvorrichtung 30 kann zunächst das eröffnete Ende des Femurs abgedichtet werden, wie anhand von Fig. 3 und 4 beschrieben. Vor der Applikation von Knochenzement wird dann die Abdichtvorrichtung 40 aufgesetzt, die Membran der Abdichtvorrichtung 30 durch eine Stichincision mittels eines Skalpells geöffnet, die Spitze der gefüllten Knochenzementkartusche an die innere Anlagefläche 48 der Abdichtvorrichtung 40 angepreßt und der Knochenzement in der z.B. in der WO-A-90/00375 beschriebenen Weise appliziert. Durch die Kombination der Abdichtvorrichtung 40 mit der Membran 30 ist es möglich, bei der Applikation von Knochenzement eine hervorragende und praktisch vakuumdichte Abdichtung des geöffneten Femurendes zu erzielen, auch wenn die Knochenfläche am geöffneten proximalen Femurende uneben ist oder einen oder mehrere Winkel bildet.

## Patentansprüche

1. Vorrichtung (10) zum Abdichten eines eröffneten Röhrenknochens, einer Knochenöffnung, einer Osteotomiefläche, eines Knochenfensters oder eines Frakturspaltes, gekennzeichnet durch eine elastische Membran in Form eines Hohlzylinders mit einem offenen und mit einem geschlossenen Ende, mit Wülsten (12,14) und/oder Verdickungen zum Abdichten von Konturen, Einziehungen, Gruben und/oder Öffnungen, wobei die elastische Membran derart vakuumdicht ausgebildet ist, dass nach dem Überstülpen der Vorrichtung über den Knochen und/oder nach dem Anpressen der Vorrichtung an den Knochen im Innern des Knochens ein Vakuum aufgebaut werden kann.

2. Vorrichtung nach Anspruch 1, wobei der freie Rand der Membran einen Wulst aufweist und/oder verdickt ist.

3. Vorrichtung nach Anspruch 1, wobei die elastische Membran Sollrisstellen, Sollperforationen und/oder Öffnungen aufweist.

4. Vorrichtung nach den Ansprüchen 1-3, wobei die elastische Membran aus Silikon besteht.

5. Vorrichtung nach den Ansprüchen 1-4, wobei die elastische Membran an ihrem freien Rand mittels einer Zugvorrichtung abdichtbar ist.

6. Vorrichtung nach einem der Ansprüchen 1-5, wobei die Zugvorrichtung elastisch oder zugfest ist.

7. Vorrichtung nach einem der Ansprüchen 1 - 6, wobei die Zugvorrichtung geführt oder fest fixiert ist.

8. Vorrichtung nach einem der Ansprüchen 1- 7, wobei die Membran Zipfel (34a), (34b) aufweist, die derart ausgebildet sind, dass sie um die Zirkumferenz des Knochens abdichtbar verknotbar sind.

9. Vorrichtung nach einem der Ansprüchen 1-8, wobei der freie Rand der elastische Membran derart ausgebildet ist, dass er aufblasbar ist, beispielsweise nach Art eines Schlauches oder Wulstes mit Gas oder Flüssigkeit, und auf diese Weise den Knochen ringförmig abdichtet.

10. Set aus Vorrichtungen nach einem der Ansprüchen 1-9, in verschiedenen Grössen zur Abdichtung verschieden grosser Knochenöffnungen.

11. Vorrichtung nach einem der Ansprüchen 1-10, in Kombination mit einer Markhöhlenabdichtvorrichtung oder einem Siegel.

## Claims

1. A device (10) for sealing an opened tubular bone, a bone opening, an osteotomy area, a bone window or a fracture fissure, characterized by an elastic membrane in form of a hollow cylinder including an open end and a closed end, with swellings (12, 14) and/or enlargements for sealing of profiles, recesses, cavities and/or openings, wherein said elastic membrane is as vacuum-tight to build up a vacuum within a bone after putting said device over a bone and/or after pressing said device to a bone.

2. The device of claim 1, wherein the free edge of said membrane includes a swelling and/or is enlarged.

3. The device of claim 1, wherein said elastic membrane includes nominal fissures, nominal perforations and/or openings.

4. The device of claims 1-3, wherein said elastic membrane is made of silicone.

5. The device of claims 1-4, wherein said elastic membrane may be sealed at its free edge by means of a drawing device.

6. The device of claims 1-5, wherein said drawing device is elastic and tensile.

7. The device of claims 1-6, wherein said drawing device is being guided or fixed.

8. The device of claims 1-7, wherein said membrane includes tags (34a, 34b), which are formed to be adapted to be sealingly tied about the circumference of the bone.

9. The device of claim 1-8, wherein said free edge of said elastic membrane is formed to be inflatable for example like a tube or swelling with a gas or a liquid, and wherein said bone being sealed by such action.

10. A set of devices according to claims 1-9 provided in different sizes to seal bone openings of different sizes.

11. The device of claims 1-10 in combination with a device for sealing a bone cavity or a seal.

## Revendications

1. Dispositif (10) pour fermer un os long ouvert, une ouverture osseuse, une surface d'ostéotomie, une fenêtre osseuse ou une fissure due à une fracture, caractérisé par une membrane élastique en forme de cylindre creux avec une extrémité ouverte et l'autre fermée, avec des renflements (12, 14) et/ou des épaississements pour étancher les contours, les retraits, les creux et/ou les ouvertures, la membrane élastique étant étanche au vide de telle sorte qu'après renversement du dispositif sur l'os et/ou après pressage du dispositif contre l'os à l'intérieur de l'os, il est possible d'y créer le vide.

2. Dispositif selon revendication 1, le bord libre de la membrane présentant un renflement et/ou étant épaissi.

3. Dispositif selon revendication 1, la membrane élastique présentant des points destinés à des fissures, des perforations et/ou des ouvertures.

4. Dispositif selon revendications 1-3, la membrane élastique étant en silicones.

5. Dispositif selon revendications 1-4, la membrane élastique pouvant être étanchée à son bord libre au moyen d'un dispositif de traction.

6. Dispositif selon revendications 1-5, le dispositif de traction étant élastique ou résistant à la traction.

7. Dispositif selon revendications 1-6, le dispositif de traction étant conduit ou immobilisé par fixation.

8. Dispositif selon revendications 1-7, la membrane présentant des cornes (34a, 34b) qui sont réalisées de telle sorte qu'elles puissent être noués de manière étanche autour de la circonférence de l'os.

9. Dispositif selon revendications 1-8, le bord libre de la membrane élastique étant réalisé de sorte à être gonflable, par exemple comme le serait un tuyau flexible ou un renflement avec du gaz ou du liquide, rendant ainsi l'os étanche en anneau.

10. Un jeu de dispositifs selon les revendications 1-9, de grosseurs différentes pour étancher les ouvertures osseuses de différentes grosseurs.

11. Dispositif selon revendications 1-10, en combinaison avec un dispositif servant à étancher un canal médullaire ou avec un sceau.
